# EUROPEAN PATENT APPLICATION

(11) **EP 4 848 299 A1**
(43) Date of publication of application: **02.12.2026**
(21) Application number: 26182120.1
(22) Date of filing: 29.05.2026
(51) Int. Cl.: G16H 20/60, A01K 29/00

(54) **PROCESS AND ELECTRONIC SYSTEM FOR DETERMINING A PERSONALIZED NUTRITIONAL PLAN FOR A PET, IN PARTICULAR A DOG**

(30) Priority: 29.05.2025 IT 202500012454
(71) Applicant: DogUp S.r.l., 35131 Padova (PD) (IT)
(72) Inventor: CECCHINI, Massimo, 35141 Padova (PD) (IT); MONGILLO, Paolo, 35142 Padova (PD) (IT)
(74) Representative: Gallo, Luca

(57) **Abstract**

Electronic system (1) for determining a personalized nutritional plan (AP) for a pet, in particular a dog, comprising: a wearable device (2), applicable to the body of a pet and provided with a motion sensor (3) for detecting motion data (DM) of the pet, a user interface (5), configured to receive basic individual parameters (PB) of the pet from a user, such as age and breed, and a memory unit (6), comprising a food database (60) in which a list of standard foods (AS) and a plurality of nutritional information (N) associated therewith are stored.

Furthermore, the electronic system (1) comprises a processing unit (7), in data communication with the wearable device (2), the user interface (5) and the memory unit (6) and configured to receive the motion data (DM) and the basic individual parameters (PB); analyze the motion data (DM) to determine the total caloric expenditure (DC) of the pet; and, based on the total caloric expenditure (DC), the basic individual parameters (PB) and the nutritional information (N) contained in the food database (60), determine a personalized nutritional plan (AP) for the pet, including indications of the quantity and frequency of intake of one or more selected foods (AF) from the list of standard foods (AS).

## Description

### Field of application

The present invention relates to a process and an electronic system for determining a personalized nutritional plan for a pet, in particular a dog, according to the preamble of the respective independent claims.

The process and the electronic system in question generally fall within the field of veterinary medicine and animal welfare, as well as within the field of the production of pet food.

The process and the electronic system are therefore advantageously intended to be used to determine, in particular in an automated manner, the optimal diet for pets, for example dogs, and to vary said diet over time so as to improve the biodiversity of the intestinal flora thereof.

### State of the art

Within the field of veterinary medicine, the nutrition of pets, and in particular dogs, is an area of growing interest for the pet food industry and among pet owners, with the aim of improving health, well-being and longevity.

In general, the diet of a dog must be balanced, complete and personalized according to numerous physiological and environmental parameters. For these reasons, determining a suitable diet for dogs is a complex process requiring the consideration of multiple factors.

In this regard, internationally recognized guidelines exist that provide indications on how to assess and satisfy the nutritional requirements of dogs, although the practical application of such guidelines may present various difficulties.

For example, the European Pet Food Industry Federation (FEDIAF), in collaboration with the Scientific Advisory Board (SAB), a panel of independent European scientific experts, periodically updates nutritional guidelines establishing the minimum and maximum requirements of macro- and micronutrients for animals according to age (puppy, adult, senior), body weight, reproductive status (pregnancy/lactation) and activity level. Such guidelines also include indications for the formulation of complete foods (covering the entire nutritional requirement) and complementary foods (to be supplemented).

Similarly, in the United States, the National Research Council (NRC) has published a detailed technical manual entitled "*Nutrient Requirements of Dogs and Cats*", which represents a worldwide scientific reference standard, containing experimental data on nutritional requirements and physiological tolerances.

Likewise, the Association for Animal Welfare Advancement (AAWA) and the American Animal Hospital Association (AAHA) promote a structured clinical approach to nutritional assessment, including periodic check-ups, evaluation of the Body Condition Score (BCS), analysis of feeding habits, and personalized recommendations. Accordingly, by following such guidelines, two main approaches are currently employed in the field of animal nutrition.

A first approach provides for producing and marketing foods, for example in the form of kibble or canned food, formulated so as to have a balance of macronutrients and micronutrients suitable for different specific groups of pets, generally distinguished according to breed and age.

The owner therefore purchases the food suitable for his or her pet depending on whether or not the pet belongs to such specific group.

However, this first approach has proved in practice not to be without drawbacks.

A first drawback lies in the fact that the nutritional plan is not necessarily suitable for the individual pet, since it does not take into account a series of factors which, according to the above-mentioned guidelines, influence the caloric requirement to be satisfied and the appropriate balance between macro- and micronutrients. Furthermore, many dietary programs based on such foods are generic and do not take into account behavioral data, individual metabolism or seasonal variations.

A further drawback lies in the fact that the owner is given no indication of the specific quantity of food to be administered to the pet and often tends to exceed the recommended maximum amount. Indeed, owners generally do not possess the expertise required to follow a balanced diet together with an appropriate physical exercise program, which are essential for weight management. For example, the growing prevalence of obesity in the canine population is indicative of this aspect.

A further drawback lies in the fact that not all products available on the market are actually of good quality or comply with the FEDIAF or NRC guidelines. Indeed, some foods may cause nutritional deficiencies or imbalances, thereby having a negative impact on the well-being of the pet.

Conversely, a second approach provides for calculating a specific nutritional plan for each pet, which satisfies all, or at least most, of the relevant factors based on the condition of the pet itself.

For example, this approach provides for using standard formulae to calculate the energy requirement of the pet and, where appropriate, correcting the obtained value by means of correction factors according to the current conditions and characteristics of the pet. Subsequently, this approach provides for determining the foods suitable for satisfying such energy requirement.

However, this second approach has also proved in practice not to be without drawbacks. A main drawback lies in the complexity of evaluating all the factors indicated in the guidelines and correctly balancing them. By way of example, the selection and quantity of dietary fiber in a dog's diet must be carefully balanced, since an excess of soluble and viscous fiber may reduce the availability of certain nutrients, thereby adversely affecting digestion and absorption.

A further drawback lies in the fact that many owners overestimate or underestimate the physical activity of the pet. Consequently, the known formulae for calculating the energy requirement, based on theoretical models and including variables related to lifestyle and other factors, still involve a risk of error that may be significant.

A further drawback lies in the difficulty of repeating such evaluations over time, due to the fact that many decisions are entrusted to the judgment and experience of the veterinary personnel responsible for evaluating the nutritional plan.

### Presentation of the invention

In this context, the problem underlying the present invention is therefore that of overcoming the drawbacks exhibited by the above-mentioned known solutions by providing a process and an electronic system for determining a personalized nutritional plan for a pet, in particular a dog, which make it possible to obtain, in a simple and efficient manner, a balanced nutritional plan according to the specific characteristics of the pet.

A further object of the present invention is to provide a process and an electronic system for determining a personalized nutritional plan for a pet, which are capable of improving and increasing the biodiversity of the intestinal flora of the pet.

A further object of the present invention is to provide a process and an electronic system for determining a personalized nutritional plan for a pet, which are versatile and can be accurately repeated.

A further object of the present invention is to provide a process and an electronic system for determining a personalized nutritional plan for a pet, which allow precise adaptation to the energy requirements of the pet.

A further object of the present invention is to provide a process and an electronic system for determining a personalized nutritional plan for a pet, which are simple and inexpensive to implement.

### Brief Description of the Drawings

The technical features of the present invention, according to the above-mentioned objects, are apparent from the content of the claims set forth below, and the advantages thereof will become more apparent from the following detailed description, given with reference to the accompanying drawings, which illustrate a purely exemplary and non-limiting embodiment thereof, wherein:
- Figures 1A and 1B show two embodiment variants of a wearable device used in the electronic system according to the invention;
- Figure 2 shows an exemplary block diagram of a preferred embodiment of the electronic system according to the invention.

### Detailed Description of Some Preferred Embodiments

With reference to the accompanying drawings, the reference numeral 1 generally indicates an electronic system for determining a personalized nutritional plan for a pet according to the invention.

Advantageously, said electronic system 1 is intended to be used for determining, in particular in an automated manner, the optimal diet for pets, for example dogs, and for varying such diet over time in such a manner as to improve the biodiversity of the intestinal flora thereof.

In the present description, a personalized nutritional plan for a dog will frequently be taken into consideration by way of example. Obviously, the features may be suitably adapted, where appropriate, for any other pet.

According to the invention, the electronic system 1 comprises a wearable device 2, configured to be applied to the body of a pet.

The wearable device 2 comprises at least one motion sensor 3 configured to detect motion data DM of the pet, comprising at least one acceleration value VA and at least one inclination value VI.

More in detail, the motion sensor 3 comprises a triaxial accelerometer 31, which is configured to detect the acceleration value VA, and a gyroscope 32, which is configured to detect the inclination value VI.

Advantageously, in order to make the detections more accurate, the motion data DM further comprise a position value VP.

Advantageously, the motion sensor 3 comprises a magnetometer 33, which is configured to detect said position value VP. In particular, the magnetometer is arranged to provide an indirect measurement of position by measuring the alignment with the Earth's magnetic field. As an alternative to, or in combination with, the magnetometer 33, a GPS module may be provided for detecting the position of the pet and enriching the behavioral profile thereof.

Obviously, by means of the values detected by the motion sensor 3 (or by a plurality of motion sensors), it is also possible to calculate indirectly other useful values, such as, for example, speed and duration of movement, according to requirements.

The wearable device 2 further comprises a communication module 4, configured to send signals corresponding to the motion data DM. For this purpose, the communication module 4 is in data communication with the motion sensor 3 in order to receive the above-mentioned motion data DM. Advantageously, the communication module 4 is a Bluetooth and/or Wi-Fi module for connection with a processing unit 7 described below, which may in particular be incorporated in a computer 10, in a smartphone, in a cloud, or in any other external device. In this manner, the wearable device 2 can advantageously transmit data in real time.

Obviously, without thereby departing from the scope of protection of the invention, the communication module 4 may alternatively or additionally comprise a USB port, which allows the wearable device 2 to be connected once it has been removed from the pet.

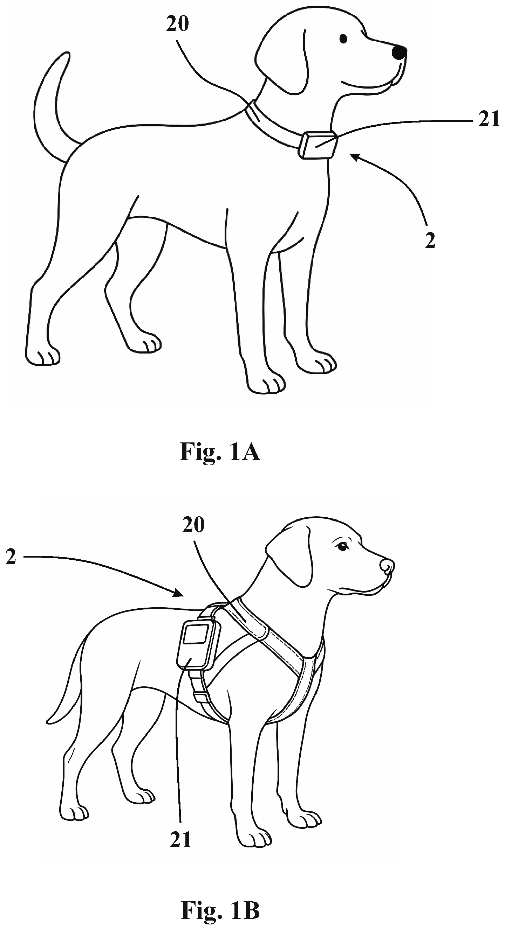

According to the preferred embodiment, the wearable device 2 further comprises fastening means 20, such as, for example, a collar or a harness, on which the motion sensor 3 and the communication module 4 are fixed, either permanently or removably, in order to facilitate application thereof to the body of the pet. Some non-limiting embodiment variants of possible configurations are illustrated, for example, in Figures 1A and 1B.

In particular, the motion sensor 3 and the communication module 4 are housed within a housing body 21 intended to be mounted on the fastening means 20.

The electronic system 1 further comprises a user interface 5, configured to receive basic individual parameters PB of the pet from a user.

The expression "basic individual parameters" is intended to mean the identifying characteristics of the pet, as well as its physiological characteristics.

More in detail, the basic individual parameters PB comprise at least an age value and breed information. Optionally, the basic individual parameters PB further comprise sex information and/or a weight value and/or reproductive status information and/or an indication of body distribution and/or a hair type.

Preferably, the basic individual parameters comprise all the above-mentioned characteristics.

In particular, the age value may be set as a precise value or selected within a range of values, for example corresponding to the life stages of the pet (for example, young, adult, etc.).

Preferably, the weight value is set as the precise value of the current weight of the pet. Furthermore, the reproductive status information advantageously relates to whether or not the pet has been neutered or sterilized and, if not, to any lactation or pregnancy status.

Furthermore, the indication of body distribution advantageously relates to the Body Condition Score (BCS) of the pet. In particular, the indication of body distribution among the basic individual parameters PB of the pet may be provided by one or more images of the pet. Alternatively, said indication of body distribution is provided as an indication selectable from at least the following: "emaciated", "very thin", "thin", "slightly underweight", "ideal", "slightly overweight", "overweight", "very overweight", "obese". In the latter case, each selectable indication is advantageously associated with a corresponding Body Condition Score (BCS).

Furthermore, the hair type advantageously relates to the length and/or texture of the hair (short, medium, long, coarse, soft).

Advantageously, the user interface 5 is further configured to receive at least one physical exercise parameter PE, relating to at least one of the following characteristics: type of activity in the absence of the owner, number of walks per day, and other types of physical activity performed with the owner.

The expression "physical exercise parameter PE" is intended to mean any information capable of defining the type and/or the amount of physical activity performed by the pet. In particular, said parameter is useful for preliminarily defining the context of the activity performed by the pet in order to improve what is detected and communicated by the wearable device 2.

More in detail, the type of activity in the absence of the owner advantageously relates to the activity that is not performed with the owner, namely whether the pet always sleeps or rests, alternates between activity and rest, or is always active.

Furthermore, the number of walks per day is advantageously taken into consideration when the walks exceed a minimum effective duration value, such as, for example, 20 minutes.

Advantageously, the user interface 5 is further configured to receive at least one health parameter PS, relating to at least one of the following characteristics: consistency of feces, appearance of the fur, amount of fur lost during seasonal changes/moulting, breath quality, frequency of night-time awakenings, emotional/psychological state, and pathologies of the pet.

The expression "health parameter PS" is intended to mean any information relating to the health condition of the pet.

In particular, the consistency of the feces is, for example, selectable from at least the following indices: "soft", "medium", "firm".

Furthermore, the appearance of the fur relates, for example, to its glossiness and is selectable from at least the following indices: "glossy", "moderately glossy", "dull". Furthermore, the amount of fur lost during seasonal changes/moulting is, for example, selectable from at least the following indices: "high", "low", "none".

Furthermore, the breath quality advantageously relates to the frequency of bad breath and is, for example, selectable from at least the following indices: "often", "sometimes", "never".

Furthermore, the emotional/psychological state advantageously relates to the emotional condition generally reflected by the activity of the pet (happy, energetic, depressed, relaxed, active, agitated, anxious, thoughtful, stressed, reactive, etc.).

Advantageously, the user interface 5 is further configured to receive at least one food preference parameter PP, relating to at least one of the following characteristics: food intolerances of the pet, foods to be excluded from the diet, and current food.

The expression "food preference parameter PP" is intended to mean any information relating to foods that the pet cannot eat or to foods that the pet prefers to eat.

Advantageously, the user interface 5 may be local or remote and may, for example, be a smartphone or a tablet, or may be implemented in the form of a mobile application allowing the display of activity graphs, personalized notifications, and/or the manual modification of the PB, PE, PS, and PP parameters of the pet.

Advantageously, a memory unit 6 is provided, comprising a food database 60 in which a list of standard foods AS and a plurality of nutritional information N associated with each standard food AS are stored.

Said standard foods AS may suitably be selected from industrially processed foods or fresh foods.

Advantageously, the standard foods AS are classified in the food database 60 as standard dry foods, such as, for example, kibble, or standard wet foods, such as, for example, canned food.

Advantageously, the food database 60 in the memory unit further comprises a list of standard nutritional supplements IS. In particular, the standard nutritional supplements IS comprise micronutrients present in smaller amounts in the standard foods AS. According to the idea underlying the present invention, the electronic system 1 comprises a processing unit 7, in data communication with said wearable device 2, said user interface 5 and said memory unit 6, and configured to receive at least the signals corresponding to the motion data DM and to the basic individual parameters PB. Furthermore, the processing unit 7 is configured to analyze the motion data DM in order to determine a total caloric expenditure DC of the pet. Preferably, said total caloric expenditure DC is determined on a daily or weekly basis.

More in detail, the processing unit 7 is configured to calculate the total caloric expenditure DC of the pet based on a physical activity classification algorithm. Said physical activity classification algorithm is advantageously configured to identify a physical activity category CF based on the motion data DM, which is related to an activity performed by the pet and to which an amount of energy consumed per unit of time QE is associated.

Furthermore, said physical activity classification algorithm is advantageously configured to determine the duration of a time interval T during which the activity associated with the physical activity category CF was performed by the pet. For example, the classification of the detected activities into physical activity categories CF (including, in particular, walking, running, playing and resting) is performed by means of an algorithm based on supervised machine learning.

The processing unit 7 is therefore configured to estimate the total caloric expenditure DC based at least on the amount of energy consumed per unit of time QE and on the duration of the time interval T.

For example, according to a particular embodiment, the caloric expenditure for each individual activity can be calculated by multiplying the amount of energy consumed per unit of time QE by the corresponding duration of the time interval T. Furthermore, the total caloric expenditure DC is advantageously given by the sum of the caloric expenditures for each individual activity.

Advantageously, the processing unit 7 is configured to analyze the motion data DM in order to determine an action category CA performed by the pet.

More in detail, the processing unit 7 is configured to determine the action category CA based on an action classification algorithm, which may be integrated into the physical activity classification algorithm. Said action classification algorithm is advantageously configured to identify an action category CA based on the motion data DM, which is related to an action performed by the pet, which, in particular, may not necessarily be related to the calculation of the total caloric expenditure DC of the pet, unless it also coincides with a physical activity category CF.

Furthermore, said action classification algorithm is advantageously configured to determine the duration of a secondary time interval TS during which the action associated with the action category CA was performed by the pet. For example, the classification of the detected actions into action categories CA (for example selected at least from: drinking, scratching, barking, and optionally others coinciding with physical activities: walking, running, playing and resting) is performed by means of an algorithm based on supervised machine learning.

According to the invention, the electronic system 1, in particular its processing unit 7, is configured, based at least on the total caloric expenditure DC, the basic individual parameters PB, and the nutritional information N contained in the food database 60, to determine a personalized nutritional plan AP for the pet, including indications of the quantity and frequency of intake of one or more selected foods AF from the list of standard foods AS in the food database 60.

Advantageously, the processing unit 7 is configured to determine and/or modify the personalized nutritional plan AP for the pet also based on the action categories CA determined by the action classification algorithm, optionally in combination with the value of the secondary time interval TS. For example, the detected actions may indicate an abnormal condition of the pet (excessive time in which the pet scratches itself, insufficient time in which the pet drinks, etc.), which requires particular adaptations of the personalized nutritional plan AP or corrective actions thereon. In this way, the use of an electronic system, which combines different motion sensors applied to the pet through a wearable device, predictive algorithms and inputs from the user, allows obtaining easily and quickly a personalized nutritional plan AP for the pet. Moreover, such personalized nutritional plan AP is extremely precise in responding to and adapting to the energy requirement of the pet.

Advantageously, the electronic system 1 further comprises a storage module 8 suitable for storing the generated personalized nutritional plan AP and/or the detected motion data DM.

Moreover, the personalized nutritional plan AP can be displayed and/or modified on the user interface 5.

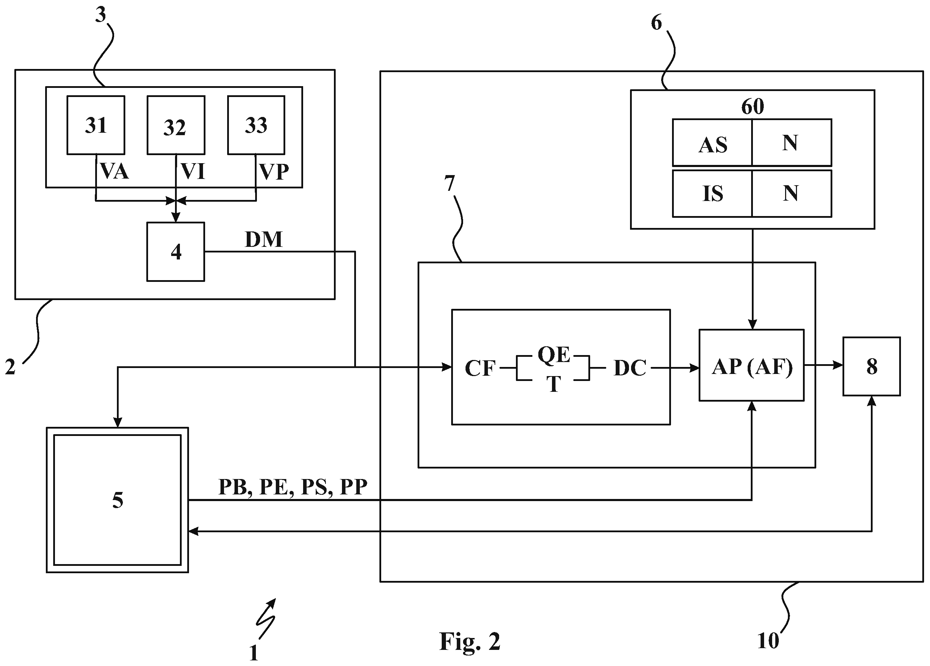

According to the preferred embodiment of the invention, schematically illustrated in figure 2, the memory unit 6 and the processing unit 7 are comprised in a single computer 10. Alternatively, the memory unit 6 is implemented in cloud. Advantageously, also the storage module 8 can be comprised in the computer 10 or implemented in cloud. For example, the storage module 8 can be a remote cloud archive in which the collected and analyzed data are stored and are accessible by the user through personal credentials.

Preferably, the processing unit 7 is configured to determine the personalized nutritional plan AP also based on at least one among the physical exercise parameter PE, the health parameter PS and the food preference parameter PP, more preferably based on all the reported parameters.

More in detail, the processing unit 7 determines the personalized nutritional plan AP by means of an algorithm based on supervised machine learning, namely by means of artificial intelligence.

In particular, such algorithm is trained and validated by setting parameters known from the literature and from case studies in which data relating to the physical activity of the animal and to its optimal diet are collected.

Appropriately, the personalized nutritional plan AP can be validated manually or automatically, for example by comparison with veterinary guidelines or other nutritional databases. Moreover, the result of the validation can be used to further train the algorithm of the processing unit 7.

Advantageously, in the case in which the indication of the body distribution of the basic individual parameters PB of the animal is provided by means of one or more images of the pet, the processing unit 7 is configured to calculate a body condition score (BCS) by means of an algorithm based on supervised machine learning starting from the images of the pet. In particular, such algorithm is trained and validated by means of predefined images associated with specific BCS values.

Moreover, the processing unit 7 is configured to determine the personalized nutritional plan AP also based on said body condition score (BCS).

According to the preferred embodiment of the invention, limit values for the quantities of foods are set in the processing unit 7.

For example, the limit values are set such that the personalized nutritional plan AP comprises a quantity comprised between 60 and 90% by weight, preferably between 70 and 80% by weight, of standard dry foods.

Moreover, the limit values are advantageously set such that the personalized nutritional plan AP comprises a quantity comprised between 10 and 40% by weight, preferably between 20 and 30% by weight, of standard wet foods.

Moreover, in the case in which standard nutritional supplements IS are also present in the food database 60, the generated personalized nutritional plan AP advantageously further comprises indications of quantity and frequency of intake of one or more standard nutritional supplements IS present in said food database 60 as a function of the age, reproductive status and/or health of the animal. For example, such supplements can be for lactating or pregnant animals or for puppies requiring a greater quantity of particular nutrients.

Advantageously, the electronic system 1 is overall configured to update the personalized nutritional plan AP, preferably automatically, at regular time intervals, for example monthly, based on the evolution of the monitored physical activity.

Moreover, at each update, the processing unit 7 is configured to remove from the personalized nutritional plan AP at least one of the selected foods AF and replace it with a different selected food AF from the list of standard foods AS.

It has in fact been surprisingly found that in this way it is possible to increase the biodiversity of the intestinal flora of the animal.

The present invention also relates to a process for determining a personalized nutritional plan AP for a pet, which advantageously uses the electronic system 1 described above, the references of which will be maintained for the sake of simplicity of description. Advantageously, therefore, the process according to the invention is intended to be used for determining, preferably in an automated manner, the optimal diet for pets, for example dogs, and for varying such diet over time in such a way that it can improve the biodiversity of the intestinal flora thereof.

The process according to the invention comprises a detection step, in which, by means of at least one motion sensor 3 present on a wearable device 2, preferably of the type described above, applied to the animal, motion data DM are detected, comprising at least one acceleration value VA and at least one inclination value VI. Preferably, the motion data DM further comprise a position value VP.

In particular, the detection step can also comprise the detection of the geographical position of the animal by means of a GPS module, for estimating the distance travelled and the environment in which the activity is carried out.

Obviously, other motion data can be detected or derived from the measured values VA, VI, VP, such as, for example, the duration of the movement and the frequency of movements.

The process further comprises a step of definition of basic individual parameters PB of the animal, comprising at least an age value and breed information. Optionally, such basic individual parameters PB further comprise sex information and/or a weight value and/or reproductive status information and/or an indication of body distribution and/or a hair type.

The expression "basic individual parameters" is intended to indicate the anagraphic characteristics of the animal, as well as its physiological characteristics.

More in detail, the basic individual parameters PB comprise at least an age value and breed information. Optionally, the basic individual parameters PB further comprise sex information and/or a weight value and/or reproductive status information and/or an indication of body distribution and/or a hair type.

Preferably, the basic individual parameters comprise all the above-mentioned characteristics.

In particular, the age value can be set as a precise value, or selected within a range of values, for example relating to the life stages of the animal (for example young, adult, etc.).

Preferably, the weight value is set as a precise value of the current weight of the animal.

Moreover, the reproductive status information is advantageously relative to whether or not the animal is castrated or sterilized and, in the case in which it is not, to the possible lactation or pregnancy status.

Moreover, the indication of body distribution is advantageously relative to the body condition score (Body Condition Score, BCS) of the animal.

Moreover, the hair type is advantageously relative to the length and/or consistency of the hair (short, medium, long, hard, soft).

Advantageously, in the definition step, at least one physical exercise parameter PE is also set, relating to at least one of the following characteristics: type of activity in the absence of the owner, number of walks per day, other types of physical activity carried out with the owner.

The expression "physical exercise parameter PE" is intended to indicate any information capable of defining the type and/or quantity of physical activity carried out by the animal. In particular, such parameter is useful for preliminarily defining the context of the activity carried out by the animal in order to improve what is detected and communicated by the wearable device 2.

More in detail, the type of activity in the absence of the owner is advantageously relative to the activity which is not carried out with the owner, namely whether the animal always sleeps or rests, alternates activity and rest, or is always active. Moreover, the number of walks per day is advantageously taken into consideration when the walks are longer than a minimum effective duration value, such as for example 20 minutes.

Advantageously, in the definition step, at least one health parameter PS is also set, relating to at least one of the following characteristics: consistency of feces, appearance of the fur, amount of fur lost during seasonal changes/moulting, breath quality, frequency of night-time awakenings, emotional/psychological state, pathologies of the animal.

The expression "health parameter PS" is intended to indicate any information relating to the health status of the animal. Moreover, the appearance of the fur is, for example, related to its shine.

Moreover, the breath quality is advantageously related to the frequency of bad smell. Moreover, the emotional/psychological state is advantageously related to the emotional condition that is generally apparent from the activity of the animal (happy, energetic, depressed, relaxed, active, agitated, anxious, thoughtful, stressed, reactive, etc.). Advantageously, in the definition step, at least one food preference parameter PP is also set, relating to at least one of the following characteristics: food intolerances of the animal, foods to be excluded from the diet, current food.

The process according to the invention further comprises a step of arranging a list of standard foods AS and a plurality of nutritional information N associated with each standard food AS.

Such standard foods AS can suitably be selected among industrially processed foods or fresh foods.

Advantageously, the standard foods AS are classified as standard dry foods, such as for example in the form of kibbles, or standard wet foods, such as for example canned food. Advantageously, the arranging step further provides for a step of arranging a list of standard nutritional supplements IS.

The process according to the invention further comprises an analysis step of the motion data DM for determining a total caloric expenditure DC of the animal.

For example, such analysis can be carried out by means of an algorithm based on supervised machine learning, as previously illustrated. In particular, the analysis step comprises the classification of the activities carried out by the animal into physical activity categories CF (including, for example: walking, running, playing, resting), based on the detected motion data DM, each of which is associated with an amount of energy consumed per unit of time QE. Moreover, the analysis step advantageously provides for determining the duration of a time interval T in which such activities associated with the physical activity categories CF are carried out. Therefore, the analysis step provides for calculating the total caloric expenditure DC based on the amount of energy consumed per unit of time QE and the duration of the time interval T. Advantageously, the analysis step can further provide for an analysis of the motion data DM for determining an action category CA performed by the animal.

For example, in this case, the analysis step provides for determining the action category CA by means of an action classification algorithm, which can be integrated into the physical activity classification algorithm. More in detail, in such analysis step, an action category CA is identified, based on the motion data DM, which is related to an action performed by the animal and, in particular, may not necessarily be related to the calculation of the total caloric expenditure DC of the animal, unless it also coincides with a physical activity category CF.

Moreover, the analysis step optionally provides for determining the duration of a secondary time interval TS in which the action associated with the action category CA was performed by the animal. For example, the classification of the detected actions into action categories CA (for example selected at least among: drinking, scratching, barking, optionally others coinciding with physical activities: walking, running, playing, resting) is carried out by means of an algorithm based on supervised machine learning. According to the invention, the process further comprises a generation step of a personalized nutritional plan AP, in which, based at least on the total caloric expenditure DC, the basic individual parameters PB and the nutritional information N of the standard foods AS, a personalized nutritional plan AP is determined for the animal, comprising indications of quantity and frequency of intake of one or more selected foods AF from said list of standard foods AS. Advantageously, moreover, the personalized nutritional plan AP also comprises suggestions relating to the frequency of daily meals and to the hourly distribution thereof.

Advantageously, in the generation step, the personalized nutritional plan AP is also generated based on at least one among the physical exercise parameter PE, the health parameter PS and the food preference parameter PP, preferably based on all the reported parameters.

Advantageously, moreover, the personalized nutritional plan AP is also generated and/or modified based on the action categories CA determined during the analysis step, optionally in combination with the value of the secondary time interval TS. For example, the detected actions may indicate an abnormal condition of the animal (excessive time in which the animal scratches itself, insufficient time in which the animal drinks, etc.), which requires particular adaptations of the personalized nutritional plan AP or corrective actions thereon.

Moreover, in the case in which standard nutritional supplements IS have also been set in the arranging step, the generated personalized nutritional plan AP advantageously further comprises indications of quantity and frequency of intake of one or more standard nutritional supplements IS as a function of the age, reproductive status and/or health of the animal. For example, such supplements can be for lactating or pregnant animals or for puppies requiring a greater quantity of particular nutrients.

Preferably, the personalized nutritional plan AP comprises a quantity comprised between 60 and 90% by weight, preferably between 70 and 80% by weight, of standard dry foods. Moreover, the personalized nutritional plan AP advantageously comprises a quantity comprised between 10 and 40% by weight, preferably between 20 and 30% by weight, of standard wet foods.

Advantageously, the process further comprises a presentation step of the personalized nutritional plan AP by means of a user interface 5 accessible remotely or locally.

Advantageously, the process further comprises a storage step, in which the collected data and the personalized nutritional plan AP are automatically saved in a storage module 8, comprising for example a remote archive, and synchronized with an associated user account.

Advantageously, the process further comprises a validation step, manual or automatic, in which the personalized nutritional plan AP is compared with veterinary guidelines or other nutritional databases.

According to the preferred embodiment of the invention, the personalized nutritional plan AP is updated, preferably automatically, at regular time intervals, for example monthly, based on the evolution of the monitored physical activity.

Preferably, at each update, at least one of the selected foods AF is removed from the personalized nutritional plan AP and replaced with a different selected food AF from the list of standard foods AS.

It has in fact been surprisingly found that in this way it is possible to increase the biodiversity of the intestinal flora of the animal.

The invention thus conceived therefore achieves the intended objects.

## Claims

1. Electronic system (1) for determining a personalized nutritional plan (AP) for a pet, in particular a dog, comprising:
- a wearable device (2), configured to be applied to the body of a pet and comprising:
- at least one motion sensor (3) configured to detect motion data (DM) of the pet, comprising at least one acceleration value (VA) and at least one inclination value (VI);
- a communication module (4), configured to send signals corresponding to said motion data (DM);
- a user interface (5), configured to receive basic individual parameters (PB) of the pet from a user, comprising at least an age value and breed information, and optionally sex information and/or weight value and/or reproductive status information and/or an indication of body distribution and/or hair type;
- a memory unit (6), comprising a food database (60) in which a list of standard foods (AS) and a plurality of nutritional information (N) associated with each standard food (AS) are stored;
- a processing unit (7), in data communication with said wearable device (2), said user interface (5) and said memory unit (6) and configured to:
- receive at least the signals corresponding to said motion data (DM) and said basic individual parameters (PB);
- analyze said motion data (DM) to determine the total caloric expenditure (DC) of the pet;
- on the basis at least of said total caloric expenditure (DC), of said basic individual parameters (PB) and of the nutritional information (N) contained in said food database (60), determine a personalized nutritional plan (AP) for the pet, including indications of quantity and frequency of intake of one or more selected foods (AF) from said list of standard foods (AS) in said food database (60);
said processing unit (7) being configured to calculate the total caloric expenditure (DC) of the pet based on a physical activity classification algorithm;
wherein said physical activity classification algorithm is configured to:
- identify a physical activity category (CF) based on movement data (DM), which is related to an activity performed by the pet and to which an amount of energy consumed per unit of time (QE) is associated;
- determine the duration of a time interval (T) in which the activity associated with said physical activity category (CF) was performed by the pet;
said processing unit (7) being configured to estimate the total caloric expenditure (DC) based at least on said amount of energy consumed per unit of time (QU) and on the duration of said time interval (T).

2. Electronic system (1) according to claim 1, **characterized in that** said at least one motion sensor (3) comprises a triaxial accelerometer (31), which is configured to detect said acceleration value (VA), and a gyroscope (32), which is configured to detect said inclination value (VI).

3. Electronic system (1) according to claim 1 or 2, **characterized in that** said motion data (DM) further comprises a position value (VP),
wherein said at least one motion sensor (3) comprises a magnetometer (33), which is configured to detect said position value (VP).

4. Electronic system (1) according to any of the preceding claims, **characterized in that** said processing unit (7) is configured to analyze the motion data (DM) to determine an action category (CA) performed by the pet based on an action classification algorithm;
said action classification algorithm being configured to:
- identify an action category (CA) based on the movement data (DM), which is related to an action performed by the pet;
- determine the duration of a secondary time interval (TS) in which the action associated with the action category (CA) was performed by the pet;
said processing unit (7) being configured to determine and/or modify the personalized nutritional plan (AP) for the pet also based on said action categories (CA) determined by said action classification algorithm.

5. Electronic system (1) according to any of the preceding claims, **characterized in that** said user interface (5) is further configured to receive:
- at least one physical exercise parameter (PE), relating to at least one of the following characteristics: type of activity in the absence of the owner, number of walks per day, other types of physical activity performed with the owner;
- at least one health parameter (PS), relating to at least one of the following characteristics: consistency of feces, appearance of the fur, amount of fur lost during seasonal changes/moulting, breath quality, frequency of night-time awakenings, emotional/psychological state, pathologies of the pet;
- at least one food preference parameter (PP), relating to at least one of the following characteristics: food intolerances of the pet, foods to be excluded from the diet, current food;
wherein said processing unit (7) is configured to determine said personalized nutritional plan (AP) also based at least on one of said physical exercise parameter (PE), health parameter (PS) and food preference parameter (PP).

6. Electronic system (1) according to claim 4, **characterized in that** the indication of the body distribution of said basic individual parameters (BP) of the pet is provided by one or more images of the pet;
wherein said processing unit (7) is configured to:
- calculate a body condition score (BCS) using an algorithm based on supervised machine learning from said images of the pet;
- determine said personalized nutritional plan (AP) also based on said body condition score (BCS).

7. Electronic system (1) according to any of the preceding claims, **characterized in that** the food database (60) in said memory unit also includes a list of standard nutritional supplements (SI);
wherein said generated personalized nutritional plan (AP) it also includes indications of the quantity and frequency of intake of one or more standard nutritional supplements (SI) present in said food database (60) based on the age, on the reproductive status and/or on the health of the pet.

8. Process for determining a personalized nutritional plan (AP) for a pet, in particular a dog, comprising the following steps:
- a detection step, in which, via at least one motion sensor (3) present on a wearable device (2) applied to the pet, motion data (DM) are detected, including at least one acceleration value (VA) and at least one inclination value (VI);
- a step of definition of basic individual parameters (PB) of a pet, including at least one age value and breed information, and optionally sex information and/or weight value and/or reproductive status information and/or an indication of body distribution and/or a hair type;
- a step of arranging a list of standard foods (AS) and a plurality of nutritional information (N) associated with each standard food (AS);
- a step of analysis of the movement data (DM) to determine the total caloric expenditure (AC) of the pet;
wherein said analysis step provides for:
- classification of the activities carried out by the pet, based on the movement data (DM), into physical activity categories (CF) to each of which an amount of energy consumed per unit of time (QE) is associated;
- determination of the duration of a time interval (T) in which the activities associated with said physical activity category (CF) are performed by the pet;
- calculation of the total caloric expenditure (DC) based on the amount of energy consumed per unit of time (QU) and the duration of the time interval (T);
- a step of generation of a personalized nutritional plan (AP), in which, based at least on said total caloric expenditure (DC), said basic individual parameters (PB), and the nutritional information (N) of said standard foods (AS), a personalized nutritional plan (AP) is determined for the pet, including indications of the quantity and frequency of intake of one or more selected foods (AF) from said list of standard foods (AS).

9. Process according to claim 8, **characterized in that** said personalized nutritional plan (AP) is updated at regular intervals based on the evolution of the monitored physical activity;
wherein, with each update, at least one of said selected foods (AF) is removed from said personalized nutritional plan (AP) and replaced with a different selected food (AF) from said list of standard foods (AS).

10. Process according to claim 8 or 9, **characterized in that** said personalized nutritional plan (AP) comprises:
- a quantity of between 60 and 90% by weight, preferably between 70 and 80% by weight, of standard dry foods;
- a quantity of between 10 and 40% by weight, preferably between 20 and 30% by weight, of standard wet foods.
